# EUROPÄISCHE PATENTANMELDUNG

(11) **EP 4 059 453 A1**
(43) Veröffentlichungstag der Anmeldung: **21.09.2022**
(21) Anmeldenummer: 22160117.2
(22) Anmeldetag: 04.03.2022
(51) Int. Cl.: A61B 17/28, A61B 17/00

(54) **CHIRURGISCHES INSTRUMENT**

(30) Priorität: 18.03.2021 DE 202021101391 U
(71) Anmelder: RZ-Medizintechnik GmbH, 78532 Tuttlingen (DE)
(72) Erfinder: Lotter, Oliver, 78549 Spaichingen (DE)
(74) Vertreter: Westphal, Mussgnug & Partner Patentanwälte mbB

(57) **Zusammenfassung**

Chirurgisches Instrument (10) mit einem ersten Gelenkteil (20) und einem zweiten Gelenkteil (30), welche um eine Drehachse (A) in einer Ebene (E) gegeneinander verschwenkbar angeordnet sind, wobei das erste Gelenkteil (20) ein erstes Griffteil (21) und ein erstes Klemmelement (22) und das zweite Gelenkteil (30) ein zweites Griffteil (31) und ein zweites Klemmelement (32) aufweist, wobei an dem ersten Klemmelement (22) quer zu der Ebene (E) eine Ablageplatte (40) angeordnet ist.

## Beschreibung

Bekannt sind chirurgische Instrumente mit einem ersten und einem zweiten Gelenkteil, welche um eine Drehachse in einer Ebene gegeneinander verschwenkbaren angeordnet sind, wobei das erste Gelenkteil am proximalen Ende ein erstes Griffteil und am distalen Ende ein erstes Klemmelement und das zweite Gelenkteil am proximalen Ende ein zweites Griffteil und am distalen Ende ein zweites Klemmelement aufweist. Derartige chirurgische Instrumente kommen in unterschiedlichsten Anwendungen zum Einsatz, beispielsweise als Fasszange, als Nadelhalter oder als Gefäßklemme.

Um an Ohren, insbesondere den Ohrläppchen, Operationen durchführen zu können, beispielsweise eingerissene Ohrläppchen zu nähen, ist es erforderlich, die Ohrläppchen zu fixieren.

Die Aufgabe der Erfindung besteht darin, ein chirurgisches Instrument bereitzustellen, mit welchem eine Operation an einem Ohrläppchen besser durchgeführt werden kann und insbesondere das Ohrläppchen verbessert fixiert werden kann.

Die Aufgabe wird erfindungsgemäß gelöst durch ein chirurgisches Instrument mit den Merkmalen des Anspruchs 1.

Vorteilhafte Ausgestaltungen und Weiterbildungen sind in den abhängigen Ansprüchen angegeben.

Das erfindungsgemäße chirurgisches Instrument mit einem ersten und einem zweiten Gelenkteil, welche um eine Drehachse in einer Ebene gegeneinander verschwenkbar angeordnet sind, wobei das erste Gelenkteil am proximalen Ende ein erstes Griffteil und am distalen Ende ein erstes Klemmelement und das zweite Gelenkteil am proximalen Ende ein zweites Griffteil und am distalen Ende ein zweites Klemmelement aufweist, zeichnet sich dadurch aus, dass an dem ersten Klemmelement quer zu der Ebene eine Ablageplatte angeordnet ist. Ein derartiges Instrument ermöglicht es, mit den Klemmelementen das Ohrläppchen derart klemmend zu halten, dass die Klemmelemente etwa unterhalb des Gehörgangs anliegen und das Ohrläppchen auf der Ablageplatte zu liegen kommt. Damit ist das Ohrläppchen einerseits durch das Instrument fixiert gehalten. Zusätzlich liegt das Ohrläppchen auf der Ablageplatte, so dass bei einem chirurgischen Eingriff die hinter dem Ohrläppchen liegende Haut des Halses geschützt ist.

Vorzugsweise weist das erste Klemmelement eine Dicke die Ablageplatte eine Länge und eine Breite auf, wobei sowohl die Länge als auch die Breite der Ablageplatte jeweils mindestens das vierfache, vorzugsweist mindestens das sechsfache der Dicke des Klemmelements beträgt. Eine derartig große Ablageplatte bietet ausreichend Platz für das Ohrläppchen.

Besonders bevorzugt weist sowohl das erste als auch das zweite Klemmelement jeweils einen ersten Klemmabschnitt, welcher in etwa in der Ebene angeordnet ist, und einen zweiten Klemmabschnitt, welcher gegen die Ebene abgewinkelt angeordnet ist, auf, wobei die Ablageplatte angrenzend an den ersten Klemmabschnitt und den zweiten Klemmabschnitt des ersten Klemmelements angeordnet ist. Ist der Klemmabschnitt abgewinkelt, kann das Instrument in unterschiedlichen Ausrichtungen relativ zum Patienten angeordnet werden, je nachdem, welche Ausrichtung für den Operateur günstig ist.

Vorzugsweise ist die Ablageplatte von dem ersten Klemmabschnitt, dem zweiten Klemmabschnitt und einer freien Kante begrenzt, wobei die freie Kante vorzugsweise gekrümmt ausgebildet ist. Dies ermöglicht eine Ausgestaltung des Instruments, welche einfach reinigbar ist und geringe Verletzungsgefahr durch Kanten oder Ritzen birgt.

Vorteilhafterweise ist der zweite Klemmabschnitt gekrümmt ausgebildet. Dadurch kann eine gute Anlage an das Ohrläppchen erreicht werden.

Gemäß einer besonders bevorzugten Weiterbildung der Erfindung weisen die Klemmelemente eine strukturierte und/oder geriffelte Klemmfläche auf. Dadurch kann die Griffsicherheit des Instruments erhöht werden.

Eine vorteilhafte Ausgestaltung der Erfindung sieht vor, dass die Klemmelemente lösbar an den Griffteilen angeordnet sind. Dies kann eine flexible Anpassung des Instruments an die anatomischen Gegebenheiten des Patienten ermöglichen.

Besonders bevorzugt weist das chirurgische Instrument eine Arretiervorrichtung zur Arretierung der beiden Gelenkteile in einer Position auf. Eine derartige Arretiervorrichtung ermöglicht die Anlage des Instruments an dem Ohrläppchen und eine Fixierung in dieser Position, ohne dass der Operateur die Klemme dauerhaft halten muss.

Vorzugsweise weist die Arretiervorrichtung einen ersten Arretierabschnitt mit mehreren Zähnen und einen zweiten Arretierabschnitt mit zumindest einem Zahn auf, wobei der erste Arrtierabschnitt an einem der beiden Griffteile und der zweite Arretierabschnitt an dem anderen der beiden Griffteile angeordnet ist, und wobei die Arretierabschnitte beim Aufeinanderzubewegen der beiden Griffteile rastend ineinandergreifen. Eine derartige Arretiervorrichtung kann konstruktiv besonders einfach realisiert werden.

Vorzugsweise sind die Griffteile gegen die Klemmelemente abgewinkelt angeordnet, wobei die Griffteile und die Klemmelemente einen Winkel zwischen 170° und 140°, beispielsweise einen Winkel von 156°, einschließen. Dadurch kann die Handhabung des Instruments vereinfacht werden.

Sind die Griffteile und die zweiten Klemmabschnitte gemäß einer vorteilhaften Weiterbildung der Erfindung in entgegengesetzte Richtungen von der Ebene abgewinkelt angeordnet, kann dies die Handhabung des Instruments weiter vereinfachen.

Ein Ausführungsbeispiel der Erfindung wird anhand der nachfolgenden Figuren ausführlich erläutert. Es zeigen
- Figur 1: eine perspektivische Ansicht eines Ausführungsbeispiels eines erfindungsgemäßen chirurgischen Instruments,
- Figur 2: eine weitere perspektivische Ansicht des chirurgischen Instruments gemäß Figur 1,
- Figur 3: eine Ausschnittvergrößerung aus Figur 2,
- Figur 4: eine Ansicht von oben auf das chirurgische Instrument gemäß Figur 1,
- Figur 5: eine Ausschnittvergrößerung aus Figur 4 und
- Figur 6: eine Seitenansicht des chirurgischen Instruments gemäß Figur 1.

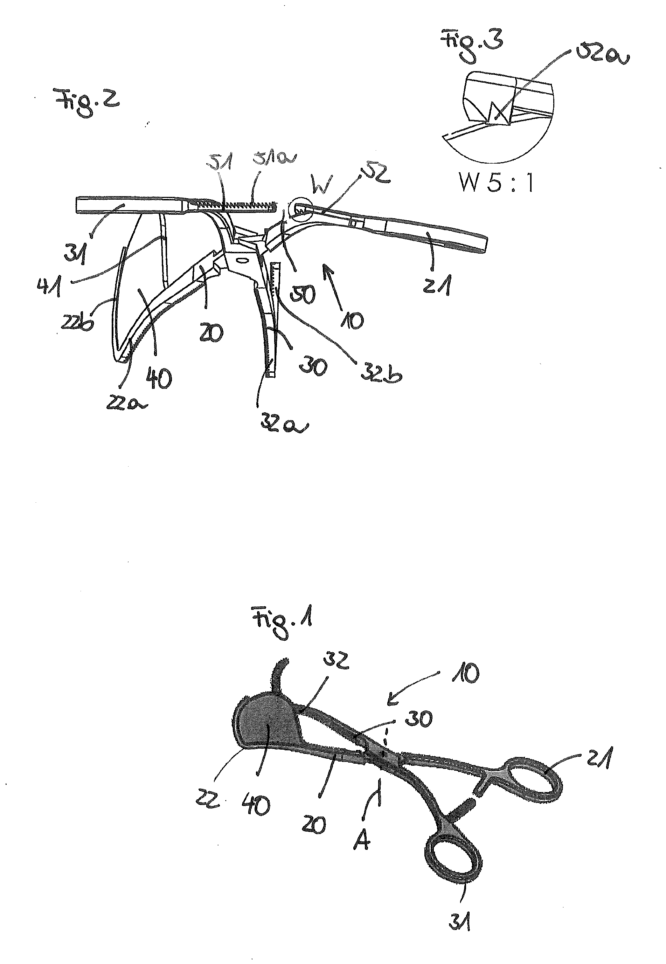

Die Figuren 1 bis 6 zeigen verschiedene Ansichten eines Ausführungsbeispiels eines chirurgischen Instruments 10. Zur besseren Übersicht sind nicht sämtliche Bezugsziffern in sämtlichen Figuren angegeben.

Das chirurgische Instrument 10 weist ein erstes Gelenkteil 20 und ein zweites Gelenkteil 30 auf, welche um eine Drehachse A in einer Ebene E gegeneinander verschwenkbar angeordnet sind. Die Ebene E ist dabei insbesondere senkrecht zur Drehachse A angeordnet. Das erste Gelenkteil 10 weist an einem proximalen Ende ein erstes Griffteil 21 und an einem distalen Ende ein erstes Klemmelement 22 und das zweite Gelenkteil 20 an einem proximalen Ende ein zweites Griffteil 31 und an einem distalen Ende ein zweites Klemmelement 32 auf.

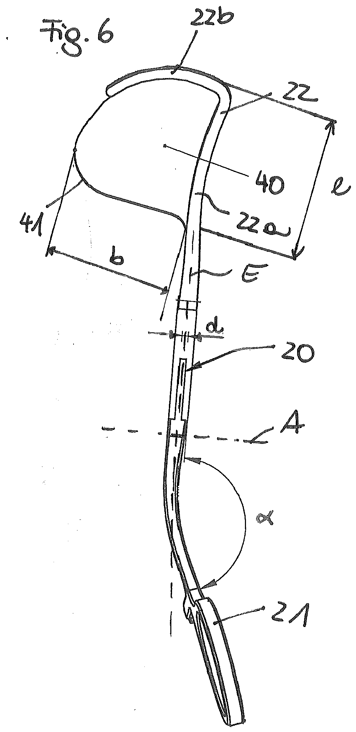

Die Griffteile 21, 31 können als Grifföse ausgebildet sein. Die Griffteile können gegen die Klemmelemente 22, 32 abgewinkelt angeordnet sein, wobei beispielsweise die Griffteile 21, 31 und die Klemmelemente 22, 32 einen Winkel α zwischen 170° und 140°, beispielsweise einen Winkel α von 156°, einschließen (vgl. insbesondere Figur 6).

Werden die Griffteile 21, 31 voneinander weg bewegt, öffnet sich vorzugsweise die Klemmung, d. h. die Klemmelemente 22, 32 bewegen sich ebenfalls voneinander weg. Werden die Griffteile 21, 31 aufeinander zu bewegt, werden auch die Klemmelemente 22, 32 aufeinander zu bewegt, um beispielsweise ein Ohrläppchen klemmend zu fixieren.

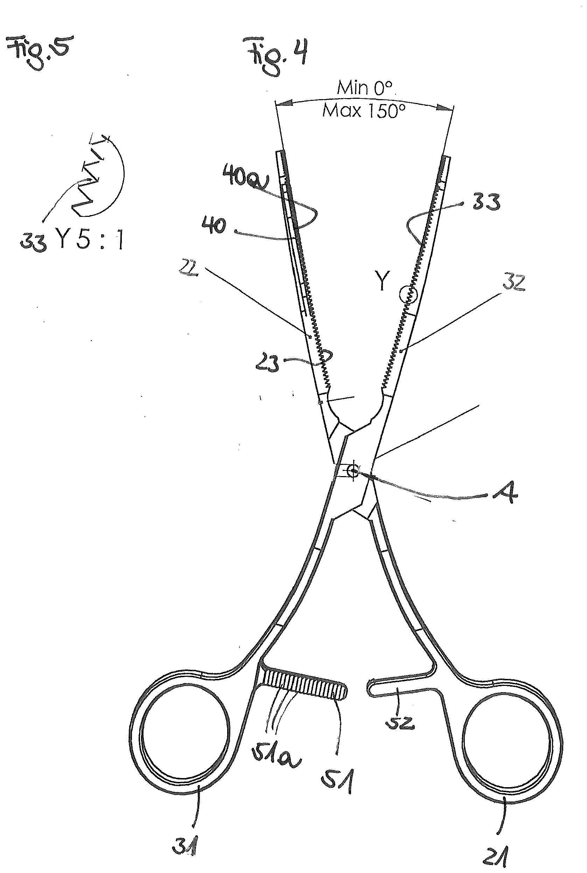

Um eine gute Fixierung vornehmen zu können, weisen die Klemmelemente 22, 32 vorzugsweise eine Strukturierung oder Riffelung 23, 33 in ihrer Klemmfläche, d. h. ihren einander zugewandten Flächen, auf (vgl. Fig. 5). Die Klemmelemente 22, 32 können lösbar an den Griffteilen 21, 31 angeordnet sein.

Das chirurgische Instrument 10 kann eine Arretiervorrichtung 50 zur Arretierung der beiden Gelenkteile 20, 30 in einer Position aufweisen. Die Arretiervorrichtung 50 kann einen ersten Arretierabschnitt 51 mit mehreren Zähnen 51a und einen zweiten Arretierabschnitt 52 mit zumindest einem Zahn 52a (vgl. Fig. 3) aufweisen, wobei der erste Arrtierabschnitt 51 insbesondere an einem der beiden Griffteile 21,31, vorliegend an dem zweiten Griffteil 32, und der zweite Arretierabschnitt 52 an dem anderen der beiden Griffteile 20, 30, vorliegend an dem ersten Griffteil 22, angeordnet ist. Beim Aufeinanderzubewegen der beiden Griffteile 21, 31 greifen die Arretierabschnitte 51, 52 vorzugsweise rastend ineinander. Dabei sind die Zähne 51a insbesondere derart ausgestaltet, dass der Zahn 52a bei der Bewegung der beiden Griffteile 21, 31 aufeinander zu die Reihe von Zähnen 51a entlang rutscht, eine Rückwärtsbewegung jedoch durch die Zähne 51a verhindert wird.

An dem ersten Klemmelement 22 ist quer zu der Ebene E eine Ablageplatte 40 angeordnet. Die Ablageplatte 40 kann flächig mit einer Anlagefläche 40a ausgebildet sein, wobei die Anlagefläche 40a insbesondere eben ausgebildet ist. Die Ablageplatte 40 kann beliebige geometrische Formen aufweisen. Wenn im Folgenden von einer Länge l und einer Breite b der Ablageplatte 40 gesprochen ist, soll darunter die größte Ausdehnung in einer Längsrichtung und die größte Ausdehnung in einer Richtung senkrecht zur Längsrichtung zu verstehen sein. Das Instrument 10 weist nur genau eine solche Ablageplatte 40 auf. An dem zweiten Klemmelement 32 ist keine solche Ablageplatte 40 angeordnet.

Das erste Klemmelement 22 kann eine Dicke d aufweisen, die insbesondere in Richtung parallel zur Drehachse A gemessen ist. Sowohl die Länge l der Ablageplatte 40 als auch die Breite b der Ablageplatte 40 betragen vorzugsweise mindestens das vierfache, vorzugsweist mindestens das sechsfache, der Dicke d des ersten Klemmelements 22 beträgt.

Sowohl das erste Klemmelement 22 als auch das zweite Klemmelement 32 können jeweils einen ersten Klemmabschnitt 22a, 32a, welcher in etwa in der Ebene E angeordnet ist, und einen zweiten Klemmabschnitt 22b, 32b, welcher gegen die Ebene E abgewinkelt angeordnet ist, aufweisen, wobei die Ablageplatte 40 angrenzend an den ersten Klemmabschnitt 22a und den zweiten Klemmabschnitt 22b des ersten Klemmelements 22 angeordnet ist. Falls die Griffteile 21, 31 gegen die Ebene E abgewinkelt sein sollten, sind vorzugsweise die zweiten Klemmabschnitte 22b, 32b in entgegengesetzte Richtung gegen die Ebene E abgewinkelt (vgl. insbesondere Figur 6).

Die Ablageplatte 40 ist insbesondere von dem ersten Klemmabschnitt 22a, dem zweiten Klemmabschnitt 22b und einer freien Kante 41 begrenzt, wobei die freie Kante 41 vorzugsweise gekrümmt ausgebildet ist. Auch der zweite Klemmabschnitt 22b kann gekrümmt ausgebildet sein. Durch die Krümmungen und Abrundungen kann sich eine gute Anpassung an die Anatomie ergeben.

Das chirurgische Instrument 10 kann derart verwendet werden, dass ein Ohrläppchen derart zwischen den zweiten Klemmabschnitten 22b, 32b fixiert wird, dass die Klemmabschnitte 22b, 32b knapp unterhalb des Gehörgangs und am oberen Rand des Ohrläppchens anliegen und das Ohrläppchen an der Anlagefläche 40a der Ablageplatte 40 anliegt.

### Bezugszeichenliste

- 10: Instrument
- 20: erstes Gelenkteil
- 21: erstes Griffteil
- 22: erstes Klemmteil
- 22a: erster Klemmabschnitt
- 22b: zweiter Klemmabschnitt
- 23: Riffelung
- 30: zweites Gelenkteil
- 31: zweites Griffteil
- 32: zweites Klemmteil
- 32a: erster Klemmabschnitt
- 32b: zweiter Klemmabschnitt
- 33: Riffelung
- 40: Ablageplatte
- 40a: Anlagefläche
- 41: freie Kante
- 50: Arretiervorrichtung
- 51: erster Arretierabschnitt
- 51a: Zahn
- 52: zweiter Arretierabschnitt
- 52a: Zahn
- A: Drehachse
- E: Ebene
- d: Dicke
- l: Länge
- b: Breite
- α: Winkel

## Patentansprüche

1. Chirurgisches Instrument (10) mit einem ersten Gelenkteil (20) und einem zweiten Gelenkteil (30), welche um eine Drehachse (A) in einer Ebene (E) gegeneinander verschwenkbar angeordnet sind, wobei das erste Gelenkteil (20) ein erstes Griffteil (21) und ein erstes Klemmelement (22) und das zweite Gelenkteil (30) ein zweites Griffteil (31) und ein zweites Klemmelement (32) aufweist,
**dadurch gekennzeichnet, dass** an dem ersten Klemmelement (22) quer zu der Ebene (E) eine Ablageplatte (40) angeordnet ist.

2. Chirurgisches Instrument nach einem der vorhergehenden Ansprüche,
**dadurch gekennzeichnet, dass** das erste Klemmelement (22) eine Dicke (d) aufweist, und dass die Ablageplatte (40) eine Länge (l) und eine Breite (b) aufweist, wobei sowohl die Länge (l) als auch die Breite (b) der Ablageplatte (40) jeweils mindestens das vierfache, vorzugsweist mindestens das sechsfache der Dicke (d) des Klemmelements (22) beträgt.

3. Chirurgisches Instrument nach einem der vorhergehenden Ansprüche,
**dadurch gekennzeichnet, dass** sowohl das erste Klemmelement (22) als auch das zweite Klemmelement (32) jeweils einen ersten Klemmabschnitt (22a, 32a), welcher in etwa in der Ebene (E) angeordnet ist, und einen zweiten Klemmabschnitt (22b, 32b), welcher gegen die Ebene (E) abgewinkelt angeordnet ist, aufweist, und dass die Ablageplatte (40) angrenzend an den ersten Klemmabschnitt (22a) und den zweiten Klemmabschnitt (22b) des ersten Klemmelements (22) angeordnet ist.

4. Chirurgisches Instrument nach einem der vorhergehenden Ansprüche,
**dadurch gekennzeichnet, dass** die Ablageplatte (40) von dem ersten Klemmabschnitt (22a), dem zweiten Klemmabschnitt (22b) und einer freien Kante (41) begrenzt ist, wobei die freie Kante (41) vorzugsweise gekrümmt ausgebildet ist.

5. Chirurgisches Instrument nach einem der vorhergehenden Ansprüche,
**dadurch gekennzeichnet, dass** der zweite Klemmabschnitt (22b) gekrümmt ausgebildet ist.

6. Chirurgisches Instrument nach einem der vorhergehenden Ansprüche,
**dadurch gekennzeichnet, dass** die Klemmelemente (22, 32) eine strukturierte und/oder geriffelte Klemmfläche aufweisen.

7. Chirurgisches Instrument nach einem der vorhergehenden Ansprüche,
**dadurch gekennzeichnet, dass** die Klemmelemente (22, 32) lösbar an den Griffteilen (21, 31) angeordnet sind.

8. Chirurgisches Instrument nach einem der vorhergehenden Ansprüche,
**dadurch gekennzeichnet, dass** das chirurgische Instrument (10) eine Arretiervorrichtung (50) zur Arretierung der beiden Gelenkteile (20, 30) in einer Position aufweist.

9. Chirurgisches Instrument nach einem der vorhergehenden Ansprüche,
**dadurch gekennzeichnet, dass** die Arretiervorrichtung (50) einen ersten Arretierabschnitt (51) mit mehreren Zähnen (51a) und einen zweiten Arretierabschnitt (52) mit zumindest einem Zahn (52a) aufweist, wobei der erste Arrtierabschnitt (51) an einem der beiden Griffteile (21, 31) und der zweite Arretierabschnitt (52) an dem anderen der beiden Griffteile (31, 21) angeordnet ist, und wobei die Arretierabschnitte (51, 52) beim Aufeinanderzubewegen der beiden Griffteile (21, 31) rastend ineinandergreifen.

10. Chirurgisches Instrument nach einem der vorhergehenden Ansprüche,
**dadurch gekennzeichnet, dass** die Griffteile (21, 31) gegen die Klemmelemente (22, 32) abgewinkelt angeordnet sind, wobei die Griffteile (21, 31) und die Klemmelemente (22, 32) einen Winkel (α) zwischen 170° und 140°, beispielsweise einen Winkel (α) von 156°, einschließen.

11. Chirurgisches Instrument nach einem der vorhergehenden Ansprüche,
**dadurch gekennzeichnet, dass** die Griffteile (21, 31) und die zweiten Klemmabschnitte (22b, 32b) in entgegengesetzte Richtungen von der Ebene (E) abgewinkelt angeordnet sind.
